(19) Europäisches Patentamt

European Patent Office

Office européen des brevets

(11) **EP 0 694 777 A2**

(12) **EUROPÄISCHE PATENTANMELDUNG**

(43) Veröffentlichungstag:
31.01.1996 Patentblatt 1996/05

(51) Int. Cl.$^6$: **G01N 3/30**, G01N 33/36

(21) Anmeldenummer: 95108280.9

(22) Anmeldetag: 31.05.1995

(84) Benannte Vertragsstaaten:
**DE FR IT NL SE**

(30) Priorität: **26.07.1994 DE 4426405**

(71) Anmelder: **Firma Carl Freudenberg**
**D-69469 Weinheim (DE)**

(72) Erfinder:
• **Schmidt, Klaus, Dr.**
**D-67663 Kaiserslautern (DE)**
• **Schmitt, Peter**
**D-67688 Rodenbach (DE)**

(54) **Vorrichtung und Verfahren zur Messung der Reissfestigkeit von textilen Flächengebilden**

(57) Eine Vorrichtung zum Messen der Reißfestigkeit textiler Flächengebilde besteht aus einem Meßsystem mit einem Elektromagneten (7), einer Masse (2), einem mit dieser Masse verbundenen, piezoelektrischen Kraftsensor (5) und einem abgerundeten Meßkörper (6). Das Meßsystem (1) trifft beim Abschalten des Elektromagneten (7) mit dem Meßkörper (6) zuerst im freien Fall auf die zu messende Textilfläche (10) auf, welche unterhalb des Meßsystems (11) waagerecht fixiert ist.

Die vom Sensor (5) aufgenommene Aufprallkraft wird als elektrostatisches Signal in einem Verstärker in ein kraftproportionales Spannungssignal umgewandelt, welches ein Rechner analog in die Reißfestigkeit umwandelt. Das entsprechende Verfahren nutzt diese Vorrichtung zu Einzel- oder auch Folgemessungen, wobei im letzteren Fall das textile Flächengebilde (10) jeweils zwischen den Einzelmessungen schrittweise unter dem Meßsystem (1) hindurchbewegt wird.

**Fig.3**

## Beschreibung

Die Erfindung befaßt sich mit einer Vorrichtung und einem Verfahren zur Messung der Reißfestigkeit von textilen Flächengebilden.

Die Bestimmung der Reißfestigkeit von textilen und anderen elastischen Flächengebilden erfolgt in der Regel nach DIN 53 857 ("Bestimmung der Höchstzugkraft"). Dabei wird die Kraft gemessen, die zum Zerreißen der Probestücke führt. Die den Flächenverbund der Probestücke zusammenhaltenden Kräfte werden dabei durch Kräfte von außen überkompensiert. Die Probe wird irreversibel zerstört.

Die Erfindung hat zur Aufgabe, eine Vorrichtung und ein Verfahren zur zerstörungsfreien Messung der Reißfestigkeit textiler Flächengebilde anzugeben.

Die Lösung dieser Aufgabe besteht in einer Vorrichtung und in einem Verfahren mit den Kennzeichen der jeweils zugehörigen Patentansprüche. Vorteilhafte Ausgestaltungen werden in den Unteransprüchen wiedergegeben.

Sinngemäß setzt sich die Vorrichtung zusammen aus einer Spannvorrichtung, welche das zu messende textile Flächengebilde bzw. deren zu messende Fläche waagerecht unter dem potentiellen Auftreffpunkt eines Fallkörper Meßsystems hält, welches sich senkrecht über der Mitte dieser Fläche befindet.

Die Erfindung wird nachfolgend anhand der Fig. 1 bis 4 näher erläutert. Es zeigen

Fig. 1 den beweglichen, frei fallenden Teil des Meßsystems,
Fig. 2 den die Masse des beweglichen Meßsystems haltenden Elektromagneten,
Fig. 3 den Meßtisch,
Fig. 4 eine Prinzipskizze der gesamten Meßanordnung mit einer bevorzugten Version der Einspann-Vorrichtungen für das zu messende textile Flächengebilde und
Fig. 5 ein typisches Kraft/Zeit-Diagramm nach einer erfindungsgemäßen Messung.

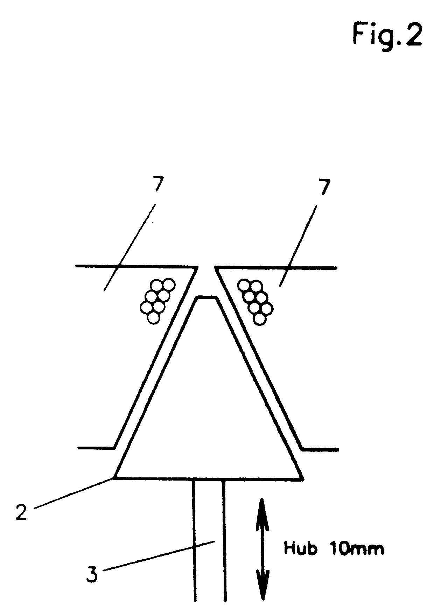

Das Meßsystem besteht, in Fallrichtung gesehen, zunächst gemäß Fig. 2 aus einem oberen, unbeweglichen Teil, gebildet durch einen ein- und ausschaltbaren Elektromagneten 7 mit einer nach unten weisenden Öffnung, welche einen konischen, noch oben hin geschlossenen Hohlraum darstellt, dessen vertikal verlaufende Längsachse 50 mm lang ist.

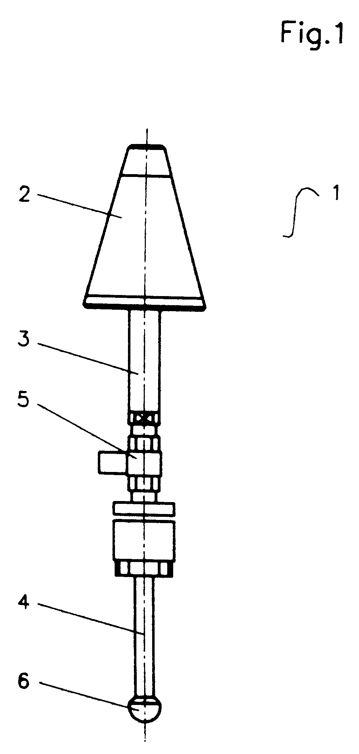

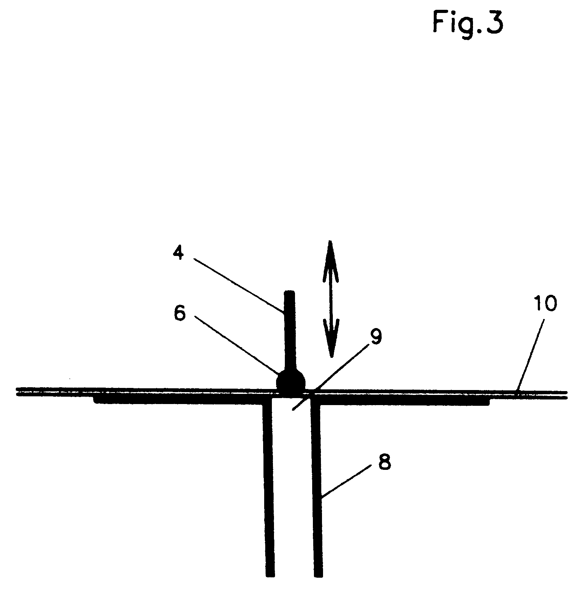

Der bewegliche Teil 1 des Meßsystems (Fig. 1 und 3) ist vertikal in Fallrichtung angeordnet und besteht in seinem oberen Teil aus einer magnetisch anziehbaren Masse 2 mit einem Gewicht von 180 bis 250 g. Diese Masse 2 ist derart nach oben zulaufend konisch geformt, daß sie in die entsprechende Öffnung des besagten Elektromagneten paßt. Die vertikal verlaufende Längsachse dieser Masse 2 soll hierzu mindestens 47 mm lang sein, um den Hohlraum des Elektromagneten 7 möglichst vollständig bezüglich dessen Länge auszufüllen.

Ein erstes, senkrechtes Bindeglied 3 mit vernachlässigbarer Masse, zum Beispiel ein Aluminiumstab, von maximal 40 mm Länge verbindet die genannte Masse 2 mit einem darunter befindlichen Kraftsensor 5.

Ein zweites, entsprechend dem ersten gestaltetes, stabförmiges, senkrechtes Bindeglied 4 verbindet den Kraftsensor 5 mit einem darunter befindlichen Meßkörper 6. Dieser Meßkörper 6 trifft auf die Mitte der zu messenden Oberfläche des textilen Flächengebildes 10 auf. Er besteht aus nicht elastischem und selbstverständlich nicht plastischem Material. Seine Masse sollte nicht mehr als die Hälfte der Masse des gesamten, frei beweglichen Meßsystems 1 betragen.

Der Meßkörper 6 besitzt eine nach unten weisende, konische oder sphärische Verrundung, deren Radius von 0,5 mm bis 25 mm beträgt.

Der Kraftsensor 5 arbeitet als piezoelektrischer Kraftaufnehmer, welcher die über das zweite Bindeglied 4 vermittelte, auf den Meßkörper 6 einwirkende Kraft bei dessen Auftreffen auf die textile Meßfläche in ein elektrostatisches Ladungssignal umwandelt. Dessen Maximum wird in einem Ladungsverstärker in ein kraftproportionales Spannungssignal mit der Einheit Volt/Newton umgewandelt und als solches einem Rechner zugeführt.

In der Regel besteht der piezoelektrische Kraftsensor 5 aus einer Meß-Unterlegscheibe, welche zwischen zwei Spezialmuttern liegt und durch einen Drehbolzen vorgespannt ist. Die Unterlegscheibe hat dadurch eine hohe Vorspannung und dementsprechend eine hohe Eigenfrequenz.

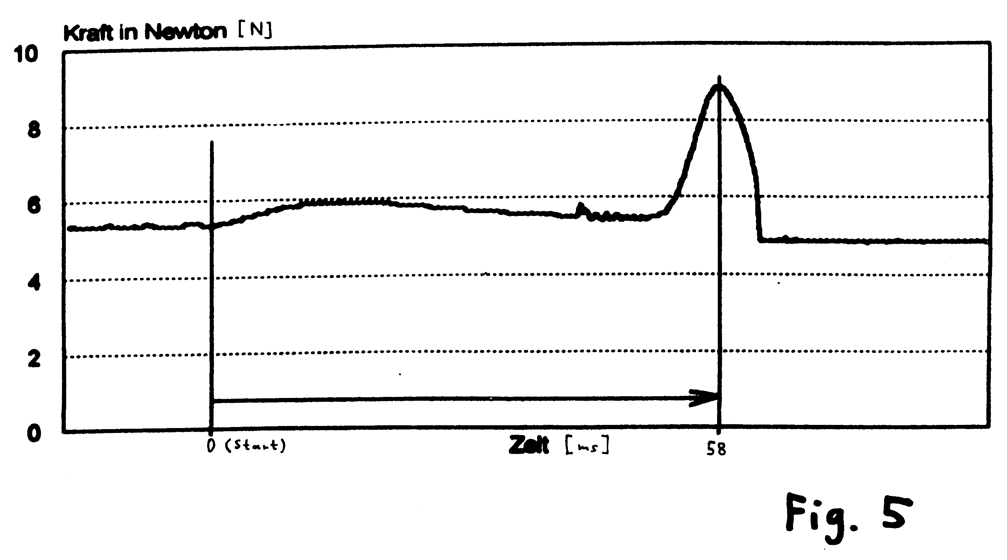

Der Rechner ermittelt die Reißfestigkeit des textilen Flächengebildes 10 folgendermaßen:
Wie aus Fig. 5 zu ersehen ist, erhöht sich bei jeder Berührung des Meßkörpers 6 mit dem zu prüfenden textilen Flächengebilde 10 der Spannungswert bis zu einem Maximum und fällt anschließend wieder ab. Der Rechner hat die Aufgabe, aus den eingelesenen Spannungswerten den Maximalwert zu bestimmen. Dieser korreliert mit der Reißfestigkeit.

Das kraftproportionale Spannungssignal steht als Einheitssignal von 0 bis 10 Volt zur Verfügung. Dies bedeutet, daß das Kraftsignal von 0 bis 20 Newton [N] mit dem Ladungsverstärker in ein proportionales Spannungssignal von 0 bis 10 Volt [V] umgewandelt wird. Dieses Spannungssignal wird im Rechner durch ein analoges Signalverarbeitungs-Programm in den entsprechenden Kraftwert umgerechnet:

$$F = 20 \text{ N}/10 \text{ V} \times U$$

F: Kraft in [N]
U: Maximal gemessene Spannung in [V]

Bei mehreren Messungen über die Warenbahn-Breite ergibt sich somit ein Profil aus Festigkeitswerten. Durch eine lineare Regressionsrechnung dieser Festigkeitswerte mit der Reißfestigkeit, die in N/5 cm angegeben wird, wird der mathematische Zusammenhang geknüpft zwischen den Maximalwerten und der Reißfestigkeit.

So ergibt sich z.B. für ein textiles Flächengebilde eine Steilheit von 52 und eine Verschiebung von -338. Die Einheit der Steilheit ist N/(5 cm × N), diejenige der Verschiebung N/5 cm. Wird nun eine Kraft von 10 N gemessen, so errechnet sich die Reißfestigkeit mit:

$$\text{Höchstzugkraft} = F \times \text{Steilheit} + \text{Verschiebung}$$

$$= 10\ N \times 52\ N / (5\ cm \times N) - 338\ N/5\ cm$$

$$= 182\ N/5\ cm.$$

Die Spannvorrichtung hält die zu messende textile Oberfläche waagerecht und zentriert 10 mm unterhalb des durch den Elektromagneten 7 über das bewegliche Meßsystem 1 fixierten Meßkörpers 6, wobei die textile Oberfläche 10 einer konstanten Spannung unterliegt.

Die Spannvorrichtung besteht hierzu aus zwei beidseitig des potentiellen Auftreffpunktes des fallenden Meßkörpers 6 befindlichen, parallel zueinander verlaufenden, länglichen Umlenkorganen, über die welche die Meßfläche des textilen Flächengebildes 10 durch beidseitigen, konstanten Zug fest spannbar ist. Die Konstanz der Einspannung ist deshalb wichtig, weil die Verformung der textilen Meßfläche 6 beim Auftreffen des Meßkörpers von dieser Spannung abhängig ist und durch den Kraftsensor 5 gemessen wird.

Zwischen den beiden Umlenkorganen und im gleichen Abstand zu diesen befindet sich der Meßtisch 8 (Fig. 3), welcher das zu prüfende textile Flächengebilde 10 unterhalb des potentiellen Aufprallpunktes des Meßkörpers 6 und zwischen den Umlenkorganen waagerecht hält. Der Meßtisch 8 ist beiderseits des potentiellen Auftreffpunktes des Meßkörpers 6 um insgesamt 20 mm unterbrochen, wodurch ein Spalt 9 gebildet wird, in den der Meßkörper 6, unter die Ebene des Meßtischs 8, eintauchen kann.

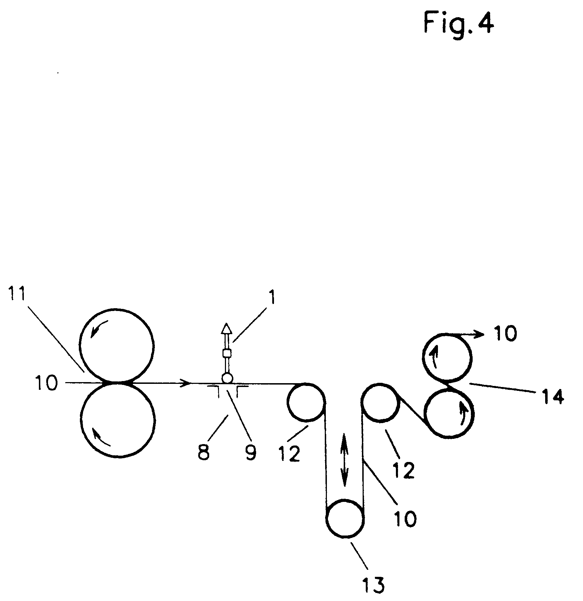

Eine vorteilhafte Variante der Vorrichtung ist in Fig. 4 gezeigt. Sie erlaubt die schrittweise Messung von Zugfestigkeiten hintereinanderliegender Flächen auf einer Textilbahn 10 beliebiger Länge in rascher Abfolge. Hierzu besteht das einlaufseitige Umlenkorgan der Spannvorrichtung aus zwei Kalanderwalzen 11, welche aufgerauhte Oberflächen besitzen und daher schlupffrei die Textilbahn 10 festhalten. Die Kalanderwalzen 11 sind gegenläufig schrittweise rotationsfähig.

Das auslaufseitige Umlenkorgan, in Transportrichtung des textilen Flächengebildes 10 gesehen jenseits des Meßtisches 8 und des Spaltes 9, wird gebildet aus einem System mit zwei horizontal voneinander beabstandeten, parallel zueinander verlaufenden, rotationsfähigen Walzen 12, über welche die Textilbahn 10 jeweils geführt wird. Zwischen diesen Walzen, vertikal beweglich, befindet sich ein Tänzer 13, welcher parallel zu den besagten beiden Walzen 12 und in einer Ebene unterhalb von diesen angeordnet ist und mindestens die dreifache Masse des beweglichen Teils des Meßsystems 1 besitzt.

Nachgeschaltet zu diesen beiden Walzen 12 mit Tänzer 13 befindet sich eine schrittweise rotationsfähige Doppelwalze 14 mit einander nicht berührenden Walzeneinheiten. Die Walzen weisen eine aufgerauhte Oberfläche auf und führen die Textilbahn 10 in S-förmiger Umschlingung schlupffrei.

Das Verfahren zur Messung der Reißfestigkeit textiler Flächengebilde verläuft unter Verwendung der Vorrichtung gemäß Anspruch 1, indem man zunächst (Fig. 1 bis 3) den beweglichen Teil 1 des Systems durch Einschalten des Elektromagneten 7 an diesem fixiert.

Danach wird das zu messende textile Flächengebilde 10 mittels der Spannvorrichtung mittig und waagerecht 10 mm unterhalb des potentiellen Auftreffpunktes des Meßkörpers 6 zentriert und dabei mit konstanter Spannung festgehalten. Der 20 mm breite Spalt 9 des Meßtisches 8 gibt dabei die zu messende Fläche für den auftreffenden Meßkörper 6 frei.

Sodann wird der Elektromagnet 7 abgeschaltet; der bewegliche Teil des Meßsystems 1 mit dem nach unten weisenden Meßkörper 6 trifft im freien Fall mit definierter Aufprallgeschwindigkeit auf die Mitte der textilen Meßfläche auf. Über den Kraftsensor 5 und den Ladungsverstärker erzeugt man durch den Aufprall ein kraftproportionales Spannungssignal, welches dem Rechner zur Ermittlung der Reißfestigkeit zugeführt wird.

Die konische Ausführung von Magnet 7 und - ihr paßgenau entsprechend - von Masse 2 erlaubt es, den Meßkörper 6 in eine bezüglich der zu messenden Textilfläche genau zentrierte, reproduzierbare Ausgangsposition zu bringen. Durch den freien Fall des beweglichen Teils 1 des Meßsystems werden Reibungskräfte und sonstige Störeinflüsse wirkungsvoll eliminiert. Nach der Messung, wenn der Meßkörper 6 auf der textilen Meßfläche aufliegt, verhindert der Hohlkonus des Elektromagneten 7 das Kippen der Masse 2, sofern die angegebenen Dimensionen für Fallhöhe, Länge der beweglichen Meßsystem-Komponente 1 und Längsachsen der Konen eingehalten werden.

Eine bevorzugte Verfahrensvariante besteht darin, aufeinanderfolgende, gleich große Flächenbereiche einer beliebig langen Textilbahn 10 schrittweise unter dem beweglichen Meßsystem 1 durchzuführen und somit jeweils eine Messung in einem benachbarten, anderen Flächenbereich durchzuführen (Fig. 4). Hierdurch ist es möglich, durch eine Vielzahl von Einzelmessungen in kurzer Zeit die - gegebenenfalls zonenweise unterschiedliche - Reißfestigkeit einer längeren Textilprobe zu bestimmen.

Hierbei wird eine Vorrichtung gemäß Anspruch 3 verwendet, wobei man während der und zwischen den Messungen das textile Flächengebilde als Bahn 10 über die ein- und auslaufseitigen Einspannstellen schrittweise führt und jeweils während der Stillstandsphase eine Fallmessung durchführt.

Das Textilbahn 10 füllt also zunächst den Kalanderspalt zwischen den Walzen 11 aus und wird dort wegen deren rauher Oberflächen festgehalten. Im weiteren Verlauf spannt sie sich auf dem Meßtisch 8 über den für den auftreffenden Meßkörper 6 bestimmten Spalt 9.

Die Textilbahn 10 wird dann weitergeführt über zwei auslaufseitige, zweckmäßigerweise bewegliche, nebeneinander befindliche Walzen 12, zwischen denen sie durch einen vertikal beweglichen, schweren Tänzer 13 nach unten gezogen wird. Diese Maßnahme ermöglicht es, eine konstante Spannung der Bahn 10 über dem Meßtischspalt 9 aufrechtzuerhalten, in hohem Maße unabhängig von dem jeweiligen Zug, welcher auf irgendeiner Seite der Meßeinrichtung etwa kurzfristig ausgeübt wird.

Im Anschluß an dieses Walzensystem führt man die Warenbahn mit S-förmiger Umschlingung um eine Doppelwalze 10 mit zwei einander nicht berührenden, gegenläufigen, rauhen Walzen. Hieran kann sich eine Aufwickelvorrichtung anschließen.

Durch schrittweises Ziehen der Textilbahn 10 über Kalander 11, Meßtisch 8, Tänzer 13 und Doppelwalze 14 werden die hintereinanderliegenden textilen Meßflächen dem fallenden Meßkörper 6 nacheinander dargeboten.

Die nach unten weisende Verrundung des Meßkörpers 6, konisch oder elliptisch, mit einem Radius von 0,1 mm bis 25 mm, wird abhängig von dem zu prüfenden textilen Material gewählt. Bei einer zweckmäßigen Fallhöhe von 10 mm soll eine elastische Verformung der textilen Meßfläche von mindestens linear 3% auftreten; d.h. der tiefste Punkt bei maximaler Verformung der Aufprallfläche auf dem textilen Flächengebilde 10 nach unten ist von seinem Ausgangspunkt vor der Messung mindestens 3% des exakten Fallweges des Meßkörpers 6 bis zum Kontakt mit der noch nicht verformten Probe entfernt. Ferner muß der Verrundungsradius selbstverständlich so groß sein, daß der Meßkörper ein - beispielsweise - grobmaschiges textiles Material nicht durchdringt, sondern lediglich stets nur zerstörungsfrei elastisch verformt.

Solche Vorversuche zur Ermittlung der geeigneter Verrundungen und Fallhöhen sind einfach durchzuführen und liefern innerhalb weniger Minuten auf empirische Weise brauchbare Parameter.

Nach deren Festlegung wird die Masse des frei beweglichen Teils 1 des Meßsystems ermittelt, wobei in Versuchsreihen festgestellt wird, bei welchen Werten die Verformung der zu prüfenden Textilfläche mindestens 3% beträgt, ohne daß das Prüfmaterial 10 zerstört wird. Auch diese Vorversuche erfordern nur wenig Zeit.

Mit der erfindungsgemäßen Vorrichtung und dem erfindungsgemäßen Verfahren, insbesondere in den vorteilhaften Ausgestaltungen, wird nicht nur eine zerstörungsfreie und rasche Messung der Reißfestigkeit einer Textilprobe ermöglicht; auch die statistische Streuung der Meßwerte durch Probenwechsel entfällt: Nach jeder Messung kann dieselbe Probe an derselben Stelle kurz darauffolgend erneut geprüft werden, indem man das vertikal bewegliche System wieder in eine reproduzierbare Ausgangslage anhebt und sofort anschließend einen weiteren, identischen Aufprall des Meßkörpers veranlaßt. Weiterhin werden durch die Ausnutzung des freien Falls Reibungseinflüsse der Vorrichtung vermieden.

Bezugszeichenliste

1   Meßsystem, beweglicher Teil
2   Masse
3   Erstes Bindeglied
4   Zweites Bindeglied
5   Kraftsensor
6   Meßkörper
7   Elektromagnet
8   Meßtisch
9   Spalt im Meßtisch
10  Textiles Flächengebilde/Textilbahn
11  Kalanderwalzen
12  Walzen
13  Tänzer
14  Doppelwalze

**Patentansprüche**

1. Vorrichtung zur Messung der Reißfestigkeit textiler Flächengebilde, bestehend aus einer Spannvorrichtung für das Flächengebilde und aus einem senkrecht darüber befindlichen Fallkörper-Meßsystem, wobei das Meßsystem besteht aus, in Fallrichtung gesehen, einem unbeweglichen, ein- und ausschaltbaren Elektromagneten (7) mit einer nach unten weisenden Öffnung, welche einen konischen, nach oben hin geschlossen Hohlraum darstellt, dessen vertikal verlaufende Längsachse 50 mm lang ist, und aus einem beweglichen Teil des Meßsystems (1), gebildet aus

- einer entsprechend der Öffnung des Elektromagneten (7) konisch nach oben zulaufend geformten, in diese Öffnung passenden, magnetisch anziehbaren Masse (2) mit einem Gewicht von 180 bis 250 g, wobei die vertikal verlaufende Längsachse dieser Masse mindestens 47 mm lang ist,
- einem ersten stabförmigen, senkrechten Bindeglied (3), welches die Masse (2) mit einem darunter befindlichen Kraftsensor (5) starr verbindet und maximal 40 mm lang ist, wobei besagter Kraftsensor (5) ein piezoelektrischer Kraftaufnehmer ist, welcher das resultierende elektrostatische Ladungssignal über einen Leistungsverstärker in ein kraftproportionales Spannungssignal umwandelt, welches einem Rechner zugeführt wird,
- einem zweiten stabförmigen, senkrechten und starren Bindeglied (4), welches den Kraftsensor (5) mit einem darunter befindlichen Meßkörper (6) verbindet und ebenfalls maximal 40 mm lang ist, wobei besagter Meßkörper (6) aus nichtelastischem und nichtplastischem, maximal die Hälfte der Masse des beweglichen Teils (1) des Meßsystems ausmachendem Werkstoff besteht und eine nach unten weisende, konische oder sphärische Verrundung aufweist, deren Radius von 0,5 mm bis 25 mm beträgt;

wobei die Spannvorrichtung die zu messende Oberfläche des textilen Flächengebildes (10) waagerecht unterhalb des am Elektromagneten (7) haftenden, beweglichen Meßsystems (1), 10 mm vom Meßkörper (6) beabstandet, in konstantem Maße gespannt hält und besteht aus

- beidseitig des potentiellen Auftreffpunktes des fallenden Meßkörpers (6) befindlichen, parallel zueinander verlaufenden, länglichen Umlenkorganen, über welche die Meßfläche des textilen Flächengebildes (10) durch beidseitigen, konstanten Zug fest spannbar ist,
- einem zwischen den Umlenkorganen, unterhalb des potentiellen Auftreffpunktes des Meßkörpers (6) und der textilen Meßfläche, befindlichen Meßtisch (8), welcher das zu prüfende textile Flächengebilde (10) waagerecht stützt und im Bereich des potentiellen Auftreffpunktes des Meßkörpers (6) auf 20 mm Länge durch einen Spalt (9) unterbrochen ist;

und wobei der Rechner das eingehende, maximale kraftproportionale Spannungssignal mit der Einheit Volt/Newton [V/N] gemäß

$$F = 20 \ N/10 \ V \times U; \qquad (F \ [N]; \ U \ [V]$$

über die gegebene Warenbahn-Breite durch eine lineare Regressionsrechnung in die Reißfestigkeit [N/5 cm] umzurechnen in der Lage ist.

2. Vorrichtung nach Anspruch 1, dadurch gekennzeichnet, daß das einlaufseitige Umlenkorgan der Spannvorrichtung aus einem eine beliebig lange Textilbahn (10) zwischen Walzen (11) mit aufgerauhten Oberflächen schlupffrei haltenden, schrittweise rotationsfähigen Kalander besteht und das auslaufseitige Umlenkorgan gebildet wird aus einem System, bestehend aus zwei horizontal voneinander beabstandeten, parallel zueinander verlaufenden, rotationsfähigen Walzen (12), zwischen welchen eine parallel zu diesen ausgerichtete, vertikal bewegliche, Tänzerwalze (13) mit mindestens der dreifachen Masse des Meßsystems angeordnet ist, und ferner bestehend aus einer diesen Walzen (12) nachgeschalteten, schrittweise rotationsfähigen Doppelwalze (14) mit aufgerauhten Oberflächen, welche die Textilbahn (10) in S-förmiger Umschlingung schlupffrei zu führen in der Lage ist.

3. Verfahren zur Messung der Reißfestigkeit textiler Flächengebilde, bei dem man unter Verwendung einer Vorrichtung gemäß Anspruch 1 das bewegliche Meßsystem (1) aus Masse (12), Kraftsensor (5) und Meßkörper (6) durch Einschalten des Elektromagneten (7) an diesem fixiert, bei der man dann die zu messende textile Fläche des Flächengebildes (10) mittels der Spannvorrichtung mittig und waagerecht 10 mm unterhalb des potentiellen Auftreffpunktes des frei fallfähigen Meßkörpers (6) zentriert und dabei unter konstanter Spannung festhält; bei dem man dann durch Abschalten des Elektromagneten (7) den Meßkörper (6) des Meßsystems (1) im freien Fall auf die Mitte der textilen Meßfläche auftreffen läßt und
bei man anschließend mittels des vom Kraftsensor (5) ausgehenden, im Ladungsverstärker in ein kraftproportionales Spannungssignal umgewandelten, maximalen Ladungssignals unter Zuhilfenahme eines Rechners die Reißfestigkeit bestimmt.

4. Verfahren nach Anspruch 3, dadurch gekennzeichnet, daß man das zu messende textile Flächengebilde in Form einer beliebig langen Textilbahn (10) in eine Vorrichtung gemäß Anspruch 2 spannt, hierüber schrittweise führt und jeweils während der Stillstandsphase eine Messung durchführt.

Fig.1

2

3

5

1

4

6

Fig.2

7    7

2

3    Hub 10mm

Fig.3

Fig. 4

Fig. 5